# EUROPEAN PATENT APPLICATION

(11) **EP 4 810 950 A1**
(43) Date of publication of application: **23.09.2026**
(21) Application number: 25221706.2
(22) Date of filing: 09.12.2025
(51) Int. Cl.: G01N 27/12

(54) **SENSOR UNIT FOR USE IN AN AIR FILTER SYSTEM AND METHOD OF MANUFACTURING THE SENSOR UNIT**

(30) Priority: 18.12.2024 EP 24220872
(71) Applicant: MANN+HUMMEL GmbH, 71636 Ludwigsburg (DE); Institut für Umwelt & Energie, Technik & Analytik e.V., 47229 Duisburg (DE); fem Forschungsinstitut, 73525 Schwäbisch Gmünd (DE)
(72) Inventor: HARENBROCK, Michael, 71636 Ludwigsburg (DE); SRADNICK, Holger, 71636 Ludwigsburg (DE); HEILMANN, Tanja, 71636 Ludwigsburg (DE); BANKODAD, Ahmed, 47229 Duisburg (DE); HAEP, Stefan, 47229 Duisburg (DE); OPIOLKA, Siegfried, 47229 Duisburg (DE); VORA, Alpesh Laxman, 47229 Duisburg (DE); EGETENMEYER, Ann-Kathrin, 73525 Schwäbisch Gmünd (DE); LANZINGER, Gloria, 73525 Schwäbisch Gmünd (DE); FREISINGER, Birger, 73525 Schwäbisch Gmünd (DE)
(74) Representative: Mann + Hummel Intellectual Property

(57) **Abstract**

The present disclosure relates to a sensor unit for use in an air filter system which serves to separate at least one harmful gas from an air flow. The sensor unit comprises a first sensor. The first sensor comprises two electrodes and a material layer which connects the electrodes. The electrical resistance of the material layer changes when the material layer comes into contact with the at least one harmful gas. The sensor unit further comprises a processing unit configured to determine a time derivative of a change in resistance of the material layer of at least the first sensor and to generate an output signal based on a function of the determined time derivative. Furthermore, the present disclosure relates to a method for manufacturing the sensor unit.

## Description

### Technical Field

The present disclosure relates to a sensor unit that can be used in in combination with an air filter. The air filter is used to separate a harmful gas such as NH3 or NO2 from an air flow. The present disclosure also relates to a method for manufacturing the sensor unit.

### Background

The operational life of a fuel cell depends to a large extent on the cleanliness of the air, which is necessary for the chemical reaction that takes place in the fuel cell to generate electrical energy. Cathode air filters are therefore used to separate harmful gases such as NH3, NO2 and SO2, which reduce the operational life of the fuel cell, from the air flow for the fuel cell. Cathode air filters with filter elements containing activated carbon to adsorb the pollutants are used for this purpose.

As the adsorbent gets loaded, complete adsorption of the target gas is no longer possible, resulting in a breakthrough of the gas through the filter. The harmful gases then enter the fuel cell. This gas breakthrough should be avoided to prevent damage to the fuel cell. The sensor unit is used to detect the gas breakthrough. If the sensor unit detects a gas breakthrough, the contaminant-loaded filter element can be replaced. This enables predictive maintenance of the air filter.

### Summary

The present disclosure is therefore based on the object to provide a sensor unit which can be used in an air filter system, which can be manufactured cost-effectively, and which enables reliable detection of harmful gases, for example, NH3 and NO2, thus providing an indication for a necessary filter element change.

This may enable predictive maintenance of the air filter system. The sensor unit may provide a warning when the saturation level of the filter element exceeds a saturation threshold. For example, the saturation threshold may be about 80%. The saturation threshold may be tailored to the application and operation mode of the air filter system.

The application of the sensor unit is not limited to fuel cell systems, but can also be used in systems facing similar challenges.

The object underlying the present disclosure is solved by the combination of features according to claim 1. Embodiments of the present disclosure can be taken from the dependent claims.

According to the present disclosure, it is provided that the sensor unit has a processing unit. The processing unit is designed to determine a time derivative of a change in an electrical resistance of the material layer of the first sensor. The processing unit then generates an output signal based on a function of the time derivative of the change in resistance of the first sensor. The output signal can be used to send a message to the operator of the air filter or to the operator of the air filter/fuel cell combination, for example, that the amount of harmful gases detected is too high and that the filter element needs to be replaced. The output signal can take a wide variety of forms. It can be acoustic, visual or in the form of a data packet, which is then transmitted to a device for further processing via a suitable interface.

The material layer of the first sensor can be a polyaniline (pani) or a metal oxide (MOx). The sensor unit can have at least a second sensor, whereby the material layer of the first sensor can be different from the material layer of the second sensor. The different sensors react differently to different harmful gases, making it easier to differentiate between the different harmful gases.

In one embodiment, the time derivative or the speed of the change in resistance for example, in the form of the time derivative of the relative change in resistance at the first sensor and/or second sensor is used. When determining the relative change in resistance, the measured resistance R is related to for example, an initial resistance R0 of the material layer. The initial resistance R0 can be the resistance that the material layer made of polyaniline or metal oxide has before initial exposure to the harmful gas. If the resistance R increases by 20% compared to the initial resistance R0 as a result of exposure to the harmful gas, for example, the relative change in resistance R/R0 assumes the value 1.2.

In the case of the harmful gas NH3, it has been found that with a time-limited exposure to the harmful gas (for example with an exposure duration of one minute or several minutes), the value R/R0 rises directly at the beginning of the exposure and falls again after the end of the exposure, but does not return to the initial value (R/R0 = 1) even after a longer period of time after the exposure. Due to this drift of the sensor signal, which adds up if the time-limited exposure is repeated several times with corresponding pauses, a simple correlation between the amount of harmful gas during the exposure and the sensor response based on the relative change in resistance is not possible. If, on the other hand, the time derivative of the relative change in resistance is used, the drift of the sensor signal does not affect the measurement results. By using the time derivative of the relative change in resistance Δ(R/R0)/Δt, the influence of the sensor signal drift observed with NH3 can be eliminated and the measurement accuracy can be improved.

If the concentration of NH3 is continuously increased from exposure to exposure, a good approximation of a linear correlation between the harmful gas quantity/harmful gas concentration and the time derivative is obtained in one embodiment. An increase in R/R0 can be recognised when the exposure duration is increased. This leads to problems if a correlation between R/R0 and the harmful gas concentration is to be established. The time derivative Δ(R/R0)/Δt, on the other hand, is independent of the exposure duration.

Another advantage of using the time derivative of the change in resistance is that mathematical compensation of the influence of relative humidity and temperature is not necessary. In practice, the time derivative of the relative change in resistance is independent of the influence of relative humidity and temperature, as both environmental parameters only change comparatively slowly in practice. The use of the time derivative of the change in resistance increases the measurement accuracy and simplifies the handling of the sensor unit.

In the case of the harmful gas NO2, it has been shown that the resistance of the polyaniline material layer also increases as a result of a temporary exposure to the harmful gas, but that the resistance remains practically constant after the end of the exposure, even after a longer waiting period, and does not drop back to R0. When re-exposed to the same concentration of harmful gas, R/R0 increases again by the same order of magnitude, so that a cascaded increase in R/R0 can be recognised. This makes it difficult to find a suitable correlation between R/R0 and the harmful gas concentration. However, if the time derivative Δ(R/R0)/Δt is used, it is easier to draw conclusions about the harmful gas concentration.

In one embodiment example, the processing unit is designed to generate the output signal depending on the condition as to whether the change in resistance of the material layer of the first sensor exceeds a threshold value S1. Only if, for example, the relative change in resistance R/R0 is greater than S1 (and other conditions may be fulfilled) does the processing unit of the sensor unit generate the output signal.

The processing unit can be designed to generate the output signal depending on the condition as to whether the time derivative of the change in resistance of the first sensor exceeds a threshold value S2. In one embodiment example, the output signal depends only on this condition. However, the generation of the output signal can also depend on further conditions, for example, on the condition that the change in resistance is greater than S1.

The processing unit can be designed to generate the output signal depending on the condition whether the time derivative of the change in resistance of the material layer of the first sensor (and optionally, also of the second sensor) falls below a negative threshold value S3. This criterion can be used to differentiate between NH3 and NO. While the resistance of the material layer of a sensor decreases again after exposure to NH3 has ended and the time derivative of the relative change in resistance therefore assumes values less than zero, the resistance remains at an increased level during exposure to NO2 and does not decrease even when the exposure has ended and NO2 is no longer present on the material layer. Accordingly, the time derivative here shows practically no negative values or only negative values that are close to zero, but whose absolute value is not greater than the absolute value of the negative threshold value S3.

In one embodiment, the output signal comprises a dynamic resistance ratio R/R_{ref} or the output signal is based on the dynamic resistance ratio R/R_{ref}, where R is the measured resistance of the material layer and R_{ref} is a dynamic baseline resistance. The dynamic baseline resistance R_{ref} depends on the time derivative of the change in resistance of the material layer, for example, on the time derivative of the relative change in resistance, and may change over time. By using the dynamic resistance ratio R/R_{ref} for the output signal or as the output signal, a drift of the sensor signal, which may also be caused by changes in relative humidity or temperature, can be avoided or significantly reduced.

The dynamic baseline resistance R_{ref} is defined as follows in an embodiment: R_{ref} corresponds to the measured and time-varying resistance R or R(t) of the material layer of a sensor, as long as the time derivative of the relative change in resistance Δ(R/R0)/Δt, with R0 as the initial resistance, is less than a start threshold value Z1. If the time derivative of the relative change in resistance Δ(R/R0)/Δt remains below this start threshold value Z1, the ratio of the measured resistance R to the dynamic baseline resistance R_{ref} is equal to 1, since the baseline resistance R_{ref} corresponds to the measured resistance R.

From the point at which the time derivative of the relative change in resistance Δ(R/R0)/Δt exceeds the start threshold value Z1, the dynamic baseline resistance R_{ref} is based on the resistance R(Z1) measured at the point at which the start threshold value Z1 is exceeded. The baseline resistance R_{ref} is now a temporary constant, so that the dynamic resistance ratio R/R_{ref} now deviates from the value 1 when the measured resistance R changes over time. As long as the value does not fall below a final threshold value Z2, the dynamic baseline resistance remains at the value R(Z1) or, more precisely, at the value R(t_{Z1}).

While the start threshold value Z1 is positive, the end threshold value Z2 is negative or less than zero. The positive start threshold value Z1 is exceeded when harmful gas exposure begins and the resistance in the material coating increases relatively quickly as a result. When the harmful gas exposure ends, the resistance of the material layer quickly decreases again, causing the value to fall below the end threshold value Z2.

In one embodiment, the start threshold value Z1 and the end threshold value Z2 are equal in magnitude. In an alternative embodiment, the ratio of the magnitudes of the start threshold value Z1 to the end threshold value Z2 is in a range from 0.5 to 2.

The dynamic baseline resistance R_{ref} corresponds again to the measured resistance R(t) as soon as the value falls below the end threshold value Z2. The ratio of R(t) to Rref is now 1 again and remains at this value until the time derivative of the relative change in resistance change Δ(R/R0)/Δt exceeds the start threshold value Z1 (again). In this case, R_{ref} would then be set to the resistance value measured at the time the start threshold value Z1 is exceeded again. If Δ(R/R0)/Δt then falls below the end threshold value Z2 again later, the measured resistance R(t) is used again as the dynamic baseline resistance Rref, with the result that the ratio R(t)/Rref is then equal to 1 again.

In one embodiment, the output signal comprises a concentration of the harmful gas (e.g. expressed in ppb or ppm), wherein the concentration is based on a calibration using the dynamic resistance R/R_{ref}.

A further object of the present disclosure, the provision of a method for manufacturing the sensor unit described above, is solved by the combination of features according to claim 5. Embodiments can be taken from the subclaims to claim 5.

The material layer of the first sensor can be subjected to a surface treatment with an acid. Insofar as the two sensors and their material layer have been manufactured from polyaniline in an identical manner, this surface treatment constitutes the difference between the two material layers. In one embodiment, the surface treatment is only carried out on the material layer of the first sensor, while no surface treatment is provided for the material layer of the second sensor. However, it is also conceivable that the material layer of the second sensor also undergoes a surface treatment that is different from the surface treatment of the material layer of the first sensor. Here too, the two material layers can differ and can provide different sensor signals when they are exposed to the same harmful gas.

For example, sulphuric acid (H2SO4), hydrochloric acid (HCl), nitric acid (HNO3), phosphoric acid (H3PO4) or sulphonic acid can be used as the acid. The surface treatment can include immersing the material layer of the first sensor in an aqueous solution of the acid. This can be a 1-molar to 10-molar solution of the acid (1 mol/l to 10 mol/l or for example, 3 mol/l to 7 mol/l).

The material layer can be immersed in the acid or the aqueous solution of the acid for 5 to 90 seconds for the surface treatment, for example, for 10 to 60 or 20 to 40 seconds. Immersion is optionally carried out without applying a potential.

To build up the material layer, aniline can be polymerised by applying a dynamic potential. Aniline can be part of an aqueous solution of an acid. For example, polyaniline may be formed in individual, superimposed films on a plate-shaped carrier on which the electrodes are arranged. In one embodiment, the electrodes are interdigital electrodes.

At least the material layer can be functionalised by adding metals, metal alloys or metal oxides (Pd, Ag, Pd/Sn, Zn) to the aqueous solution. For example, it is possible for the material layer of the first sensor to have at least one functionalised film, which the material layer of the second sensor does not have.

For chemical metal deposition, the material layer can be immersed in a dispersion comprising a solvent and metal nanoparticles.

### Brief Description of Drawings

The present disclosure is explained in more detail with reference to the embodiments shown in the drawing. It is shown in:
FIG. 1 shows a schematic diagram of an air filter and a fuel cell;
FIG. 2 shows the air filter with a first sensor and a second sensor;
FIG. 3 shows the first sensor with two electrodes and a material layer of polyaniline;
FIG. 4 shows the variation of exposure to the harmful gases NH3 and NO2;
FIG. 5 shows the variation of the relative change in resistance over time caused by the exposure to harmful gases in FIG. 4;
FIG. 6 shows the variation of the time derivative of the relative change in resistance caused by the exposure to harmful gases in FIG. 4;
FIG. 7 shows a further variation of exposure to the harmful gas NH3;
FIG. 8 shows the variation of the relative resistance change caused by the exposure to harmful gas in FIG. 7;
FIG. 9 shows the variation of the time derivative of the relative change in resistance caused by the exposure to the harmful gas in FIG. 7;
FIG. 10 shows the variation of a dynamic resistance ratio based on the exposure to harmful gas in FIG. 7;
FIG. 11 shows a further variation of exposure to the harmful gas NO2; and
FIG. 12 shows the variation of a dynamic resistance ratio based on the exposure to harmful gas in FIG. 11.

### Detailed Description

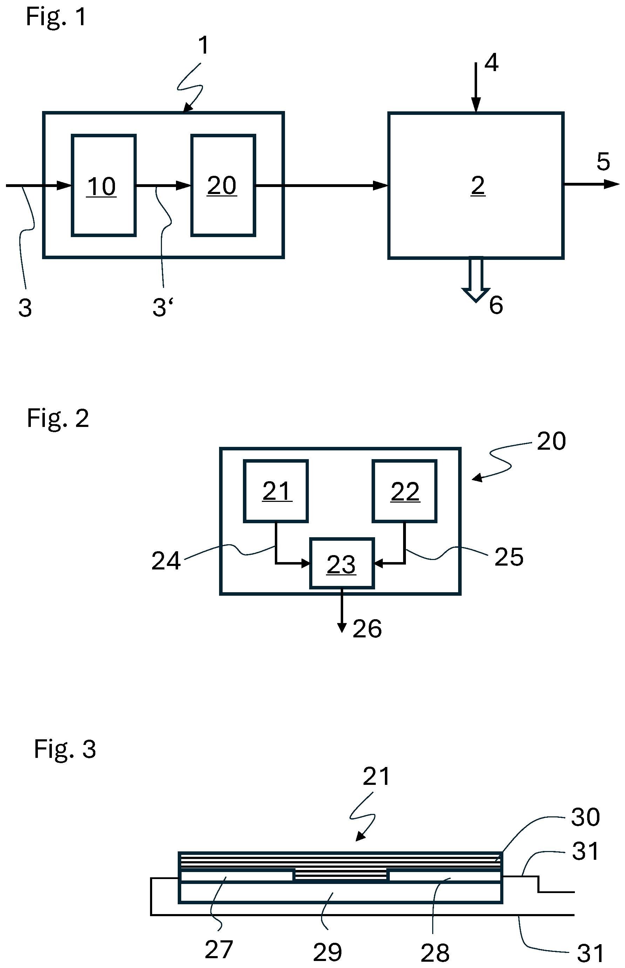

FIG. 1 shows an air filter system 1 and a downstream fuel cell 2. The air filter system 1 comprises an air filter with filter element 10 and a sensor unit 20. An air flow 3 enters the air filter system 1 and passes through the air filter 10. Harmful gases such as nitrogen dioxide (NO2) and ammonia (NH3) and/or sulfur dioxide (SO2) are separated from the air flow 3 by the air filter 10, which comprises adsorbent material. The cleaned air flow 3' now passes the sensor unit 20, which is used to check the effectiveness of the air filter 10. The sensor unit 20 is designed to detect the harmful gases (e. g., NO2, NH3 and/or SO2) in the ppm range and also in the ppb range. The cleaned cathode air flow 3' enters the fuel cell 2, where the oxygen in the air flow 3' reacts with an energy carrier such as hydrogen 4 to form water 5, thereby generating electrical energy 6.

FIG. 2 schematically shows the sensor unit 20. The sensor unit 20 comprises a first sensor 21, a second sensor 22 and a processing unit 23. In the processing unit 23, signals 24, 25 from the sensors 21, 22 are processed into an output signal 26. The output signal 26 can, for example, be transmitted to a further processing device (not shown) via a suitable interface. The output signal 26 can then be used, for example, to obtain the information that a harmful gas concentration determined is too high and that the filter element 10 must be replaced as a result.

FIG. 3 schematically shows the structure of the first sensor 21. The structure of the second sensor 22 corresponds to the structure of the first sensor 21, so that only the structure of the first sensor 21 is discussed. The sensor 21 has a first electrode 27 and a second electrode 28, which are arranged on a plate-shaped carrier 29 made of glass. For example, the electrodes are made of gold.

The sensor 21 also has a material layer 30 made of polyaniline, which electrically connects the two electrodes 27, 28. The material layer 30 comprises individual layers or films. Polyaniline is an electrically conductive polymer. When the material layer 30 comes into contact with NH3 or NO2, which may be contained in the air flow 3', the electrical conductivity of the material layer changes, so that the resistance between the first electrode 27 and the second electrode 28 changes. The change in resistance can be measured via electrical lines 31.

The material layer 30 of the first sensor 21 differs from the material layer 30 of the second sensor 22 in that the first sensor 21 with the material layer 30 is immersed in an aqueous solution of sulphuric acid for a certain period of time. This immersion in the aqueous solution changes the properties, for example, reactive properties, of the material layer 30 of the first sensor 21 compared to the untreated material layer of the second sensor 22.

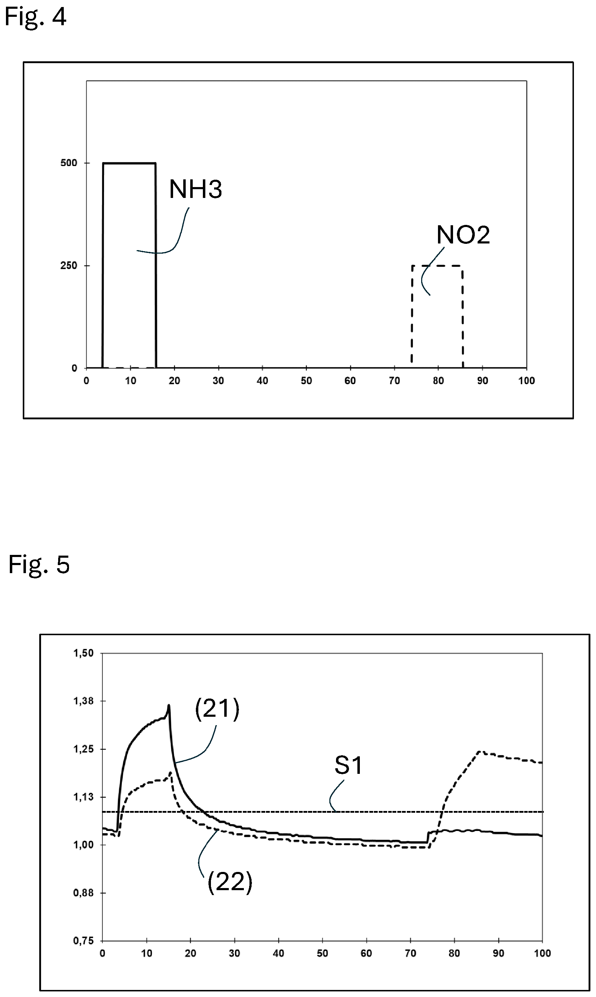

For test purposes, the sensor unit 20 is exposed to the harmful gas NH3 and, with a time delay, to the harmful gas NO2. The diagram in FIG. 4 shows the selected concentration in ppb, the exposure duration (10 min) and the time interval (60 min) between the first exposure to NH3 and the second exposure to NO2. The air flow 3' is guided to the two sensors 21, 22 with an NH3 concentration of 500 ppb and, after a break of 60 minutes, with an NO2 concentration of 250 ppb.

The solid line in the diagram in FIG. 5 corresponds to the reaction of the first sensor 21. The dashed line corresponds to the sensor reaction of the second sensor 22. The time in minutes is plotted on the x-axis and the relative change in resistance R/R0 on the y-axis, where R0 corresponds to the initial resistance of the material layer of the two sensors 21 and 22 before exposure to the harmful gas. It can be seen that the first sensor 21, which was post-treated with H2SO4, shows a stronger reaction when exposed to NH3 than the second sensor 22, which was not post-treated with H2SO4. After exposure to NH3, the sensor signal of the two sensors 21 and 22 returns to at least substantially the same level.

When exposed to NO2, the sensor 21 post-treated with H2SO4 shows practically no reaction, while the untreated sensor 22 shows a clear reaction (the relative change in resistance R/R0 increases to approx. 1.25). After the increase to the value 1.25, however, the relative change in resistance remains at this level and does not return to anywhere near the original value of 1.0. A threshold value S1 for the relative change in resistance R/R0 is shown as an example in the diagram in FIG. 5. The threshold value S1 here is approx. 1.1. While this threshold value is reached by both sensors when NH3 is applied, only the sensor signal of the untreated sensor 22 is above this threshold value S1 when NO2 is applied.

Based on the comparison with the threshold value S1 and the sensor signal of the two sensors 21, 22, a statement could be made in real operation as to whether the sensor unit has been exposed to NH3 or NO2. If both the first sensor 21 and the second sensor 22 show a sensor response above the threshold value S1, it can be concluded that the harmful gas is NH3. The processing unit can thus generate a corresponding output signal.

If, on the other hand, only the second sensor 22 shows a reaction above the threshold value S1, it can be concluded that the harmful gas is NO2. The processing unit would now generate a correspondingly different output signal.

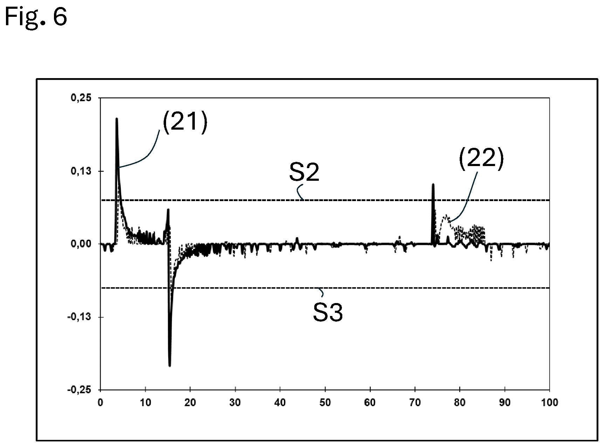

FIG. 6 shows the course of the time derivative of the relative resistance change Δ(R/R0)/Δt over time. As in FIG. 5, the corresponding sensor response as a result of the exposures shown in FIG. 4 is also shown here.

FIG. 6 clearly shows that a bipolar pulse can be assigned to both sensors when exposed to NH3. Both the first sensor (post-treated with H2SO4) and the second sensor 22 show a pulse peak at the beginning of the NH3 exposure. When this exposure ends, a negative pulse peak can be recognised, which corresponds approximately to the absolute amount of the initial positive pulse peak.

Exposure to NO2, on the other hand, only results in a unipolar pulse. A negative pulse peak at the end of the NO2 exposure is not recognizable.

In the diagram in FIG. 6, a second threshold value S2 is shown as an example for the positive pulse peaks and a third negative threshold value S3 for the negative pulse peaks. The amount of the threshold values can be the same or different. If both sensors 21, 22 now each detect a bipolar pulse, whereby the respective positive pulse peak is above the second threshold value S2 and the respective negative pulse peak is below the third threshold value S3, this is an indication of the harmful gas NH3. By checking the corresponding conditions, the processing unit can generate the output signal that can be derived from this.

If the sensor signal from the two sensors 21, 22 does not have a negative pulse peak (condition relating to threshold value S3 not fulfilled) and at the same time the condition relating to the relative resistance change R/R0 is met, i.e. the sensor signal from the second sensor 22 is greater than S1 and the sensor signal from the first sensor 21 is less than S1, the processing unit determines an output signal that indicates the presence of NO2.

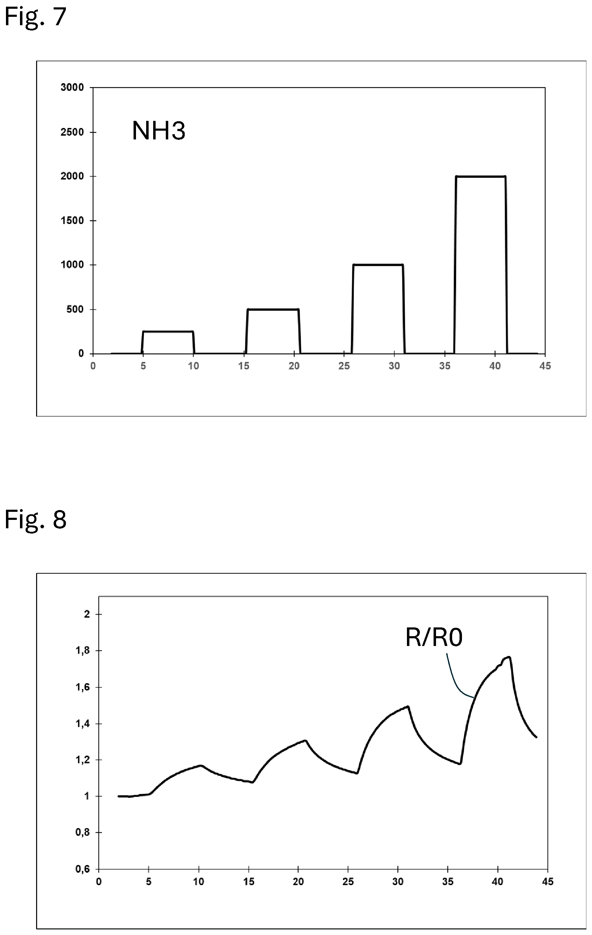

FIG. 7 shows the variation of exposure of a polyaniline sensor to NH3. Starting at a concentration of 250 ppb, the gas concentration is gradually increased to 500, 1000 and 2000 ppb. Between the individual stages of harmful gas exposure, each lasting 5 minutes, there are breaks of 5 minutes.

The resistance of the polyaniline sensor changes as a result of exposure to harmful gases, as shown in FIG. 7.

FIG. 8 shows the relative resistance change R/RO over time.

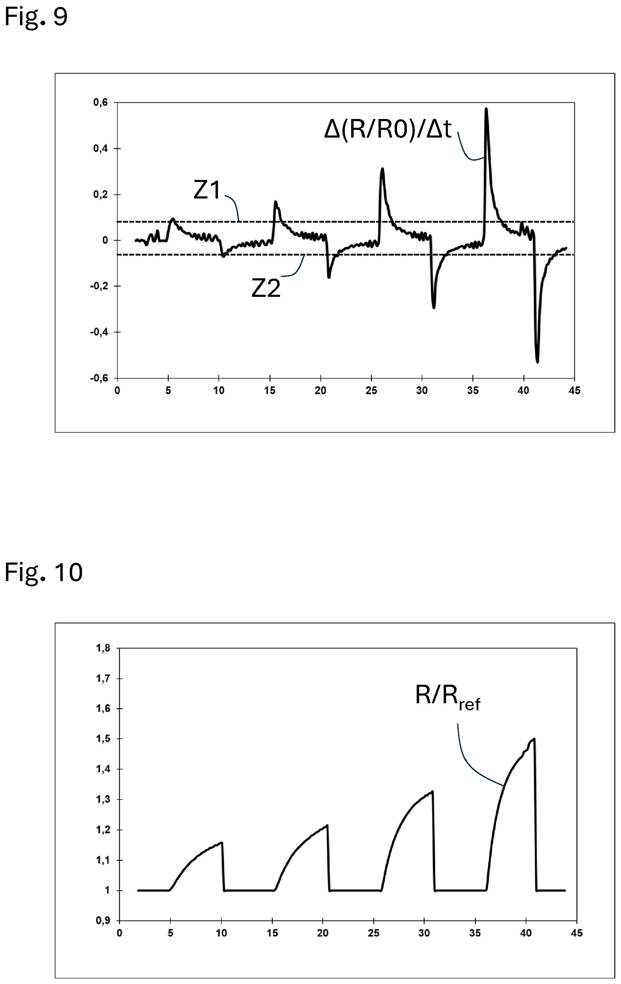

FIG. 9 shows the time derivative of the relative change in resistance in FIG. 8 (Δ(R/R0)/Δt). Due to the first stage of exposure to harmful gases, the time curve of Δ(R/R0)/Δt exceeds a start threshold value Z1 at approximately 5 to 6 minutes (see upper dashed line in FIG. 9). When the first stage of exposure ends after approximately 5 minutes, the time derivative of the relative change in resistance Δ(R/R0)/Δt drops to values below zero and falls below a final threshold value Z2 (see lower dashed line in FIG. 9). The start threshold value Z1 is between 0 and 0.2 s⁻¹, while the final threshold value is less than zero. In the second stage of harmful gas exposure (approx. between 15 and 20 minutes), the start threshold value Z1 is first exceeded and then the final threshold value Z2 is again undershot. Exceeding the start threshold value Z1 and falling below the end threshold value Z2 is repeated in the third and fourth stages of harmful gas exposure.

FIG. 10 shows the variation of a dynamic resistance ratio R/Rref. The numerator of the dynamic resistance ratio is the resistance measured by the sensor. The denominator corresponds to a dynamic baseline resistance R_{ref}, which is determined by Δ(R/R0)/Δt and the threshold values Z1 and Z2. The dynamic baseline resistance R_{ref} may be set and reset based on the time derivative Δ(R/R0)/Δt and the threshold values Z1 and Z2.

As long as the start threshold value Z1 has not been exceeded starting from time t = 0, the dynamic baseline resistance R_{ref} corresponds to the measured resistance R(t). Accordingly, until the point in time when the time curve of Δ(R/R0)/Δt exceeds the value Z1 for the first time, the dynamic resistance ratio R/R_{ref} equals 1. After exceeding the start threshold value Z1, the dynamic resistance ratio increases to approx. 1.15. In this phase from approx. t = 5 min to approx. t = 10 min, the dynamic baseline resistance R_{ref} is constant and corresponds to the resistance determined by the sensor at the intersection of Z1 with the time curve Δ(R/R0)/Δt. At the point in time at approx. t = 10 min, when the value falls below the end threshold value Z2, the dynamic baseline resistance is again equated with the measured resistance R(t), whereby the ratio R/R_{ref} returns to the value 1. Due to the repeated exceeding of Z1 and the subsequent falling below Z2 (see FIG. 9), the dynamic resistance ratio R/R_{ref} (see FIG. 10) is reset to the value 1 after each stage of harmful gas exposure. The dynamic resistance ratio R/R_{ref} can be correlated with the values of the harmful gas exposure (see FIG. 7), so that based on this correlation and the temporal course of the dynamic resistance ratio R/R_{ref}, the sensor unit can display specific values for the harmful gas concentration.

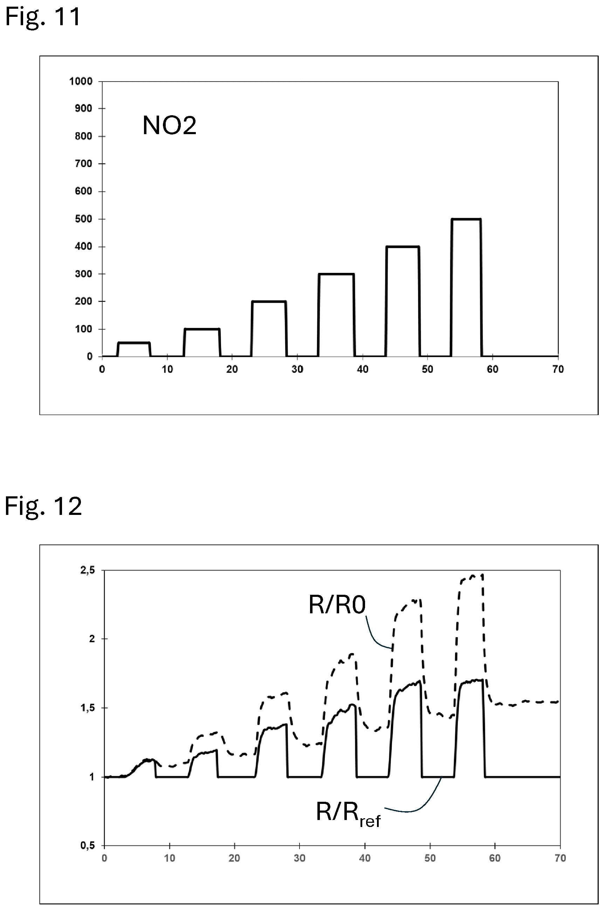

FIG. 11 shows another time curve of harmful gas exposure with NO2. A sensor with a metal oxide layer is exposed to six stages with different concentrations ranging from 50 to 500 ppb. FIG. 12 shows the time curve of the relative resistance change R/RO (dashed line) and the time curve of the dynamic resistance ratio R/R_{ref}, whereby the value R/R_{ref} was calculated in analogy to the embodiment shown in FIG. s 7 to 10. The use of R/R_{ref} for the output signal allows sensor drift to be compensated. It has also been shown that the use of R/R_{ref} can effectively compensate for possible influences of changes in relative humidity and temperature.

Determining the time derivative of the relative change in resistance enables a better and more accurate statement to be made about the harmful gases detected. Due to the usually slowly changing temperature and humidity conditions, mathematical compensation of the sensor signals is not necessary when using the time derivative Δ(R/R0)/Δt. This results in a simple sensor unit with high measurement accuracy. Due to the surface treatment of the material layer of only one sensor with an otherwise identical structure of the material layer of both sensors, the processing unit of the sensor unit can differentiate between NH3 and NO2.

According to various embodiments, the sensor unit can comprise more than two sensors, for example three, four or a larger array of sensors, each having material layers with different compositions and/or surface treatments. By providing multiple sensors with deliberately varied sensitivities and selectivities to NH3, NO2 and other harmful gases, the processing unit can evaluate a richer pattern of resistance changes and time derivatives. This enables improved gas discrimination, more robust detection under varying environmental conditions, and enhanced redundancy. If one sensor drifts or fails, the remaining sensors can still provide reliable information about harmful gases.

### List of Reference Numbers

- 1: air filter system
- 2: fuel cell
- 3: air flow (3' clean air flow)
- 4: hydrogen
- 5: water
- 6: energy
- 10: air filter
- 20: sensor unit
- 21: first sensor
- 22: second sensor
- 23: processing unit
- 24: sensor signal
- 25: sensor signal
- 26: output signal
- 27: first electrode
- 28: second electrode
- 29: carrier
- 30: material layer
- 31: line

## Claims

1. Sensor unit (20) for use in an air filter system (1) which serves to separate at least one harmful gas from an air flow (3), the sensor unit (20) comprising:
a first sensor (21)comprising
two electrodes (27, 28), and
a material layer (30) connecting the two electrodes (27, 28), wherein an electrical resistance of the material layer (30) changes when the material layer (30) comes into contact with the at least one harmful gas; and
a processing unit (23) configured to determine a time derivative of a change in an electrical resistance of the material layer (30) of the first sensor (21), and further configured to generate an output signal (26) based on a function of the determined time derivative.

2. Sensor unit (20) according to claim 1, wherein the material layer (30) is formed from polyaniline or metal oxide.

3. Sensor unit (20) according to claim 1 or 2, further comprising:
a second sensor (22) comprising
two further electrodes, and
a further material layer connecting the two further electrodes,
wherein the material layer (30) of the first sensor (21) is different from the further material layer of the second sensor (22),
wherein the processing unit (23) is further configured to determine a time derivative of a change in an electrical resistance of the further material layer of the second sensor (22), and further configured to generate the output signal further based on a function of the determined time derivative of the second sensor (22).

4. Sensor unit (20) according to claim 3, wherein the processing unit (23) is further configured to identify the at least one harmful gas based on comparing the respective electrical resistances of the material layer (30) of the first sensor (21) and the further material layer of the second sensor (22).

5. Sensor unit (20) according to one of claims 1 to 4, wherein the sensor unit (20) is configured to generate the output signal (26) based on whether the change in electrical resistance of the material layer (30) of the first sensor (21) exceeds a threshold value S1.

6. Sensor unit (20) according to one of claims 1 to 5, wherein the sensor unit (20) is configured to generate the output signal (26) based on whether the time derivative of the change in electrical resistance of the material layer (30) of the first sensor (21) exceeds a threshold value S2.

7. Sensor unit (20) according to one of claims 1 to 6, wherein the sensor unit (20) is configured to generate the output signal (26) based on whether the time derivative of the change in electrical resistance of the material layer (30) of the first sensor (21) falls below a negative threshold value S3.

8. Sensor unit (20) according to one of claims 1 to 7, wherein the output signal (26) is based on a dynamic resistance ratio R/R_{ref}, wherein R is a measured electrical resistance of the material layer (30) and R_{ref} is a dynamic baseline resistance of the material layer (30) that depends on the determined time derivative of the change in electrical resistance of the material layer (30).

9. Sensor unit (20) according to claim 8, wherein the dynamic baseline resistance R_{ref} is defined as follows:
R_{ref}= measured electrical resistance R(t), as long as the time derivative of the relative change in resistance Δ(R/R0)/Δt with R0 as the initial resistance, is smaller than a start threshold value Z1;
R_{ref}= measured electrical resistance R(Z1) at the point at which the time derivative of relative change in resistance Δ(R/R0)/Δt exceeds the start threshold value Z1 and as long as the time derivative of relative change in resistance Δ(R/R0)/Δt does not fall below an end threshold value Z2; and
R_{ref}= measured resistance R(t) as soon as Δ(R/R0)/Δt falls below the end threshold value Z2.

10. Sensor unit (20) according to claim 8 or 9, wherein the output signal (26) is indicative of a concentration of the at least one harmful gas, wherein the concentration is determined based on a calibration with the dynamic resistance ratio R/R_{ref}.

11. Method for producing a sensor unit (20) according to one of claims 1 to 10,
wherein the material layer (30) of the first sensor (21) is subjected to a surface treatment with an acid.

12. Method according to claim 11, wherein at least one of sulphuric acid (H2SO4), hydrochloric acid (HCl), nitric acid (HNO3), phosphoric acid (H3PO4) and sulphonic acid is used as the acid.

13. Method according to claim 11 or 12, wherein the surface treatment comprises immersing the material layer (30) of the first sensor (21) in a 1-molar to 10-molar aqueous solution of the acid.

14. Method according to one of claims 11 to 13, wherein aniline in an aqueous solution of an acid is polymerized by applying a dynamic potential to build up the material layer (30).

15. Method according to claim 14, wherein the material layer (30) is functionalized by mixing at least one of metals, metal alloys and metal oxides into the aqueous solution.

16. Method according to claim 14 or 15, wherein for chemical metal deposition, the material layer (30) is immersed in a dispersion comprising a solvent and metal nanoparticles.
